(19) Europäisches Patentamt
European Patent Office
Office européen des brevets

(11) **EP 2 628 456 B1**

(12) **EUROPEAN PATENT SPECIFICATION**

(45) Date of publication and mention
of the grant of the patent:
**12.08.2015 Bulletin 2015/33**

(51) Int Cl.:
***A61B 17/225*** (2006.01)

(21) Application number: **12155616.1**

(22) Date of filing: **15.02.2012**

(54) **Shock wave therapy device with dynamic target tracking**

Stoßwellentherapievorrichtung mit dynamischer Zielnachführung

Dispositif de thérapie d'onde de choc avec suivi de cible

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**21.08.2013 Bulletin 2013/34**

(73) Proprietor: **Dornier Med Tech Systems GmbH 82234 Wessling (DE)**

(72) Inventor: **Bohris, Christian 82152 Krailling (DE)**

(74) Representative: **Grünecker Patent- und Rechtsanwälte PartG mbB Leopoldstraße 4 80802 München (DE)**

(56) References cited:
**EP-A1- 1 757 244      DE-A1- 10 260 594
US-A- 5 419 327        US-A1- 2003 149 352**

• NEICU T ET AL: "Synchronized moving aperture radiation therapy (SMART): Average tumour trajectory for lung patients", PHYSICS IN MEDICINE AND BIOLOGY, TAYLOR AND FRANCIS LTD. LONDON, GB, vol. 48, no. 5, 7 March 2003 (2003-03-07), pages 587-598, XP002614578, ISSN: 0031-9155, DOI: 10.1088/0031-9155/48/5/303 [retrieved on 2003-02-18]
• WILBERT JÜRGEN ET AL: "Tumor tracking and motion compensation with an adaptive tumor tracking system (ATTS): System description and prototype testing", MEDICAL PHYSICS, AIP, MELVILLE, NY, US, vol. 35, no. 9, 7 August 2008 (2008-08-07), pages 3911-3921, XP012116223, ISSN: 0094-2405, DOI: 10.1118/1.2964090

**Description**

The invention concerns a shock wave therapy device with a shock wave therapy source that is configured to emit a shock wave having a focus onto a target into a body of a patient, with an imaging device, which is configured to visualize the target, with a control unit, which is configured to move the target relative to the emitted shock wave, with a respiration detector, which is configured to detect the respiration of the patient and configured to produce a respiration signal.

In shock wave therapy, body concrements such as urinary stones or gall-stones are disintegrated by means of focused shock waves. Other forms of shock wave therapy are the treatment of pain, the treatment of closed vessels, the treatment of the cardiac muscle, etc. in a corresponding area.

The shock waves can be generated, with an extracorporeal source, outside the patient and are focused on a target within the body. Focusing can be achieved, for example, by use of an acoustic lens.

The focus is often characterized by its, so-called, -6dB zone, i.e. the zone, where the maximum pressure of the shock wave is higher than half of the focal maximum value. Typically, this can be described by an ellipsoid with a long axis along the direction of propagation of the shock wave and short axes in the lateral plane that is perpendicular to the axial direction. The lateral - 6dB focal width ranges between 3 and 15 mm for different lithotripters; the axial focal width is typically about 10 times larger.

In order to direct the focus of the shock wave source at the concrement or target, within the body, image modalities using both X-ray or ultrasound are common.

If the position and the orientation of the image plane relative to the shock wave focus is known and if the shock wave focus is contained within the image plane, the shock wave focus can be presented as a mark on the diagnostic image.

In practice, mechanical devices which link the image device with the shock wave source are common. For example, DE 39 15 384 C2 describes a mechanical arm with a holder for an ultrasound transducer which allows to image the patient from various angles but forces the transducer to be oriented such that the shock wave focus is at the center line of the image plane.

Related prior art is also disclosed in "Ultrasound Monitoring of Kidney Stone Extracorporeal Shockwave Lithotripsy with an with an External Transducer: Does Fatty Tissue Cause Image Distortions That Affect Stone Comminution?"; C.Bohris, et al.; J. Endourology 24, 81-88, 2010.

The user can thus move the patient, for example, with the aid of a motorized patient stretcher, relative to the shock wave source, such that the shock wave focus can be aligned to the stone being imaged.

Typical shock wave treatment implies the application of e.g. 3000 shock waves which are delivered with a frequency of 1 to 2 Hz.

However, the position of the concrement is subjected to respiratory motion. For example, the kidney movement is mainly in the cranio-caudal direction. Ultrasound scanning of the organ movement showed that the motion is quasi-sinusoidal. For reference Davis et al is cited. (Davis S. et al. Ultrasound quantitation of respiratory organ motion in the upper abdomen, J.Br. Radiol 67:1096-1102, 1994.) "There is a sharp rise in organ displacement occurring, on average, within 1 s during inspiration, followed by more shallow fall during expiration with a slight pause at end-expiration. The average length of the respiration cycle was 4.4 s."

This study reported the maximum displacement of the kidney distance to be about 11 mm +/- 4 mm with quiet respiration. Deep breathing resulted in organ movement of about 40 mm. Deep breathing was also investigated by L. Schwartz et al. (Kidney mobility during respiration. Radiotherapy and Oncology 32:84-86, 1994.) with similar results.

In shock wave therapy, breathing is strongly dependent on the anaesthetic protocol.

Since the cardinal heading of the kidney is cranio-caudal and the cardinal heading of shock wave propagation is perpendicular to that, the kidney movement is along the shorter lateral axis of the shock wave focus. Depending on motion amplitude and stone size, respiratory motion may therefore result in a significant number of shock waves, that miss the target, and thus in a significant reduction in stone disintegration.

At a distance of about 5 mm between focus and stone centre, the decrease in disintegration effect is typically not significant. However, at about 10 mm, the disintegrative effect by the shock waves is divided in about half. If even greater distances are to be recognized, the effect rapidly deteriorates. It is again referred to by C. Bohris in J. Endourology 24, 81-88, 2010.

Related prior art is also disclosed for instance in DE 3146628 C3, DE 10260594 A1, DE 3943644 C2, DE 3811872 A1, EP 0460536 B1, US 2003/0149352 A1, DE 4241161 A1, DE 3900893 A1, DE 3736733 A1, DE 3743883 C3 and DE 10 2007 004334 A1.

One approach to avoid shock waves, which miss the target, but may cause medical side-effects, is to trigger shock wave release, is disclosed in DE 3146628 C3.

There are various strategies proposed to discriminate between the situation that the target coincides with the shock wave focus, or does not.

There are respiratory monitors available which translate the respiratory motion into an electric signal. Examples are respiratory belts which are fixed around the abdomen. Due to breathing, its length increases which can be measured by inductive, resistance, pressure or other techniques.

Alternatively, chest electrodes, as used for ECG monitors, can be employed for electric impedance measurement which is a function of the lung volume.

Alternatively, electric temperature sensors can be introduced to the nostrils or, in the case of a general anaesthesia, in the tubus.

DE 3621935 C2 reveals a lithotripter with a respiratory monitor and adjustable thresholds which can be used for triggering.

DE 10260594 A1 reveals a therapeutic shock wave device with a respiratory monitor, an imaging device and a correlation unit, which supports the user to yield a set point of the time function of the respiratory monitor which correlates to the target being in the shock wave focus. This set point can be used to trigger the shock wave release.

DE 3943644 C2 and DE 3811872 A1 reveal therapeutic shock wave devices with an ultrasound imaging device for continuous imaging. By utilizing image processing methods, it is monitored, if the stone is within the shock wave focus. This is used to trigger shock wave application.

US 5 065741 A further discloses an extra corporeal ultrasonic lithotripter with a variable focus.

In EP 0460536 B1 a therapeutic shock wave device is introduced which emits low amplitude sub-therapeutic test pulses. The echoes of the test pulses are collected. If the echo amplitudes are above a certain level, therapeutic shockwaves are released.

The disadvantage of triggering the shock wave release is the introduction of short periodic treatment interruptions, which are time intervals not used for treatment. In addition, patients with a low sedation may avoid painful shock waves by evasive movements.

Another approach, in order to enable a continuous shock wave release, is tracking the stone. Those systems were introduced in US 2003/0149352 A1, DE 4241161 A1, DE 3900893 A1, DE 3736733 A1, DE 3743883 C3, DE 102007004334 A1. These systems employ image processing methods applied to ultrasound imaging devices of the therapeutic shock wave system to detect the target position, for example, the stone position, relative to the shock wave focus. This is used to control mechanical drives either in the lithotripter or the patient stretcher, such that the focus is directed to the target.

US2003/0149352 A1 discloses a therapeutic shock wave device which provides a real-time, automatic tracking system for stones, so that the instant stone position of a patient can be localized and adjusted for in real-time.

Stone detection by image processing is challenging due to various reasons:

Ultrasound image quality is often poor in practice due to speckle and image artifacts. Most commonly, 2-dimensional imaging is employed, such that the stone may leave the image plane with deep breathing or patient movements. In addition, therapy intends to induce changes to the target, e.g. stone fragmentation, such that the target undergoes changes, which make it hard to track. Therefore, those systems tend not to be robust in practice.

The object of the invention is to provide a therapeutic shock wave device which keeps the target area in the shock wave focus during the respiratory cycle, is easy to use, is reliable and allows for a continuous shock wave release.

This object is solved by a shock wave therapy device according to claim 1, further comprising a control unit configured to control the relative movement between the target and shock wave focus in accordance with a predefined movement pattern.

By movement patterns, paths of motion and/or motion sequences are to be understood. Differently to those solutions, where the shock waves are only emitted when a target is within a predefined area, the relative movement between target and focus compensates for the respiratory motion of the target and allows such a continuous shock wave application. This is similar to those solutions where the position of the target is continuously tracked. However, the current invention makes a more simple and robust, though still very precise operation possible.

A stable patient position is assumed in the beginning. Using an imaging modality, the target is visualized and, with positioning means, e.g. electric motors of a patient stretcher, aligned to the focus. Due to respiratory motion, the target moves relative to the focus in a quasi-periodic way.

Such a quasi-periodic movement resembles such a periodic function that does not meet a strict mathematical function, but has a high similarity to it.

The time between two succeeding breaths of a patient may vary. The amplitude of each breath also may vary from one to the other. Over time, a kind of fuzziness thus remains concerning the exact form of the respiration motion.

The target position $P(t) = (P_x(t), P_y(t), P_z(t))$ relative to the focus is a function of time. The invention is founded on the assumption, that this function can be approximated by a successive series of identical partial functions. The partial function $R(t) = (R_x(t), R_y(t), R_z(t))$ represents the target position during a single respiratory cycle. The device provides a user interface such that this partial function can be adapted by the user to the respiratory cycle. The user may select a function from a stored set of patterns, which can be further optimized by setting one or more parameters $p_1, p_2, p_3, ...$

Pattern functions may be deduced from empirical data obtained e.g. from ultrasound image sequences obtained during previous representative shock wave therapy treatments. Pattern functions may be attributed to certain breathing conditions, e.g. artificially driven breathing with an inserted tube or spontaneous breathing. Parameters $p_1$, $p_2$, $p_3$,...may include the maximum amplitude and times for inspiration and expiration phases. The optimized function is $R_p(t) = R(t, p_1, p_2, p_3,)$.

The user may also employ imaging devices in order to adapt the parameters.

For example, a sequence of breaths can be diagnosed with an ultrasonic monitor.

The user may deduce an average value of the maximum amplitude of the respiratory motion. Similarly, the average time periods for inhaling and expiration can be estimated. The resulting motion pattern defined by $R_p(t)$ represents a so-called counter movement to the targets' breath related movement, so that the target remains approximately stable relative to the shock wave focus.

To keep the target in the vicinity of the shock wave focus, the device performs relative motions between the target and shock wave source, which are the same as $R_p(t)$.

For instance, motors of the patient stretcher are moved with a velocity, which can be deduced from the time derivative of the function $R_p(t)$. That way the target is moved but the shock wave source remains stationary.

Alternatively motors may move the therapy head and the patient remains stationary. The focus of the shock wave source may also be shifted in space by electronic steering using a shock wave source as described in US 5065741 A.

The motion is triggered by the output signal of a respiration detector. Whenever the detector shows, for example, an inspiration, the relative motion is started.

The signal amplitude attained by the respiration detector is not necessarily straight proportional to the lateral distance between the target, such as a kidney and the shock wave focus. For instance, the abdominal girth when an abdominal belt is used is not linearly connected to a caudal-cranial movement of the kidney.

However, from the rising edge of the signal amplitude, a trigger signal indicating the start of the inhalation phase may be deduced.

The user can monitor and optimize the compensation of the respiratory motion by the imaging modality. Under visual control, the pattern function or its selected parameters may be optimized. In addition, the user may optimize the triggering of the motion start by adjusting the trigger level.

The advantage of the invention is that the target is tracked and interruptions by shock wave blockage are avoided. The procedure is robust and safe, since it can be monitored and controlled continuously by the user. Embodiments of advantage or covered by the dependent claims are detailed in the following.

Imaging modality is preferably an ultrasound system integrated into the shock wave source. Typically the target, e.g. a urinary stone, can be visualized such that the target remains within the 2D image plane through the respiratory cycle. The orientation of the integrated ultrasound transducer defines the cardinal direction of motion. It is possible that the control unit comprises a mechanical drive unit, which is configured to move the target relative to the emitted shockwave

It is of advantage if a user interface is comprised, which is configured to select a stored, predefined or preselected movement pattern. If a storage device is comprised, in which one or more movement patterns are stored, a kind of library can be used, to refer back to previously acquired patient information or, information resembling the patient, having a target which shall actually be treated. The movement pattern may concern a predefined movement pattern.

It is advantageously if the preselected movement pattern is a movement pattern, based on empirical data which was acquired previous to the operation of the shock therapy device 1.

It is also of advantage if movement of the target is choosable by way of a manual selector, such as a button or a control device.

Another embodiment is characterized in comprising a function generator, which is configured to select a certain movement pattern depending on the respiration signal. If the function generator is configured to select the predefined movement pattern automatically, the shock wave therapy device can be used even by inexperienced personnel and the treatment, conducted by the shock wave therapy device according to the invention, can be shortened.

It is advantageously if the height of the amplitude of the movement of the target and/or the duration of an inhalation related movement, and/or the length of an expiration related movement, and/or a curve progression of the movement are choosable, preferably steplessly or via selecting predefined values.

It is also of advantage, if the device is having an override button to chose a predefined movement pattern, resembling a movement of the target, which ensures the least deviation from a movement of a target suitable for a multitude of patients, so that the target remains in spite of movement due to breathing stable relative to the shock wave focus.

Another embodiment concerns a motion control unit, being configured to cause one or more motors such as to move a patient stretcher or to move the shock wave therapy source such as a therapy head. For instance, the patient on the patient stretcher can be moved in the first of the two alternatives ways and the target, included in the patient, is in consequence, also moved. By such a configuration, movement can be easily applied indirectly to the target. The two possibilities to cause relative movement of the target can be combined. In another embodiment the focus itself is controlled by electronic steering.

If the shock wave therapy device additionally comprises a monitor suitable to display an ultrasound image of a target area including a target, the position of the target relative to the shock wave focus can be visually controlled and adequate measures can be taken to keep the target in the focused shock wave.

The invention is also detailed in some Figures:

Figure 1     discloses a schematic layout of the shock wave therapy device according to the invention.

Figure 2     discloses an ellipsoid resembling the focal volume, for example the -6 dB isobar, due to the omitted shock wave. The shock wave source itself is not shown in this figure.

Figure 3     discloses the lateral displacement movement of a target, for instance a kidney relative to the focus, due to respiratory motion.

Figure 4     discloses one exemplary movement pattern, with a linear movement.

Figure 5     discloses another example of movement pattern, as an alternative to a path of motion shown in Figure 4.

Figure 6     discloses a more detailed exemplary of a single movement pattern, as shown in Figure 5.

Figure 7     discloses the interaction of the respiration detection signal, the trigger signal for the shockwave and the movement pattern.

Figure 8     discloses the interaction of the movement of the target due to respiratory motion and the forced countermovement, to keep the target approximately stable.

The figures are only of schematic nature and intended for clarification of the invention only and not intended to limit the scope of protection. The same reference numbers are used for the same elements.

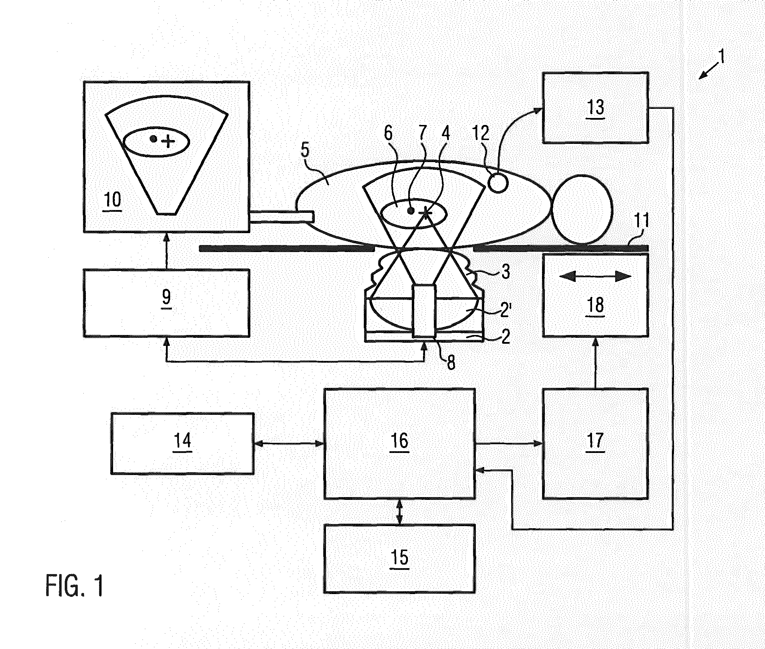

In Figure 1, a shock wave therapy device 1 is disclosed. The shock wave therapy device 1 comprises a therapy source 2, configured to emit a shock wave, which is focused by an acoustic lens 2'. The therapy source 2, may comprise an electromagnetic shock wave emitter. Via a coupling cushion, acoustical coupling to the patient 5 is provided. The patient 5 is positioned on a patient stretcher 11. The coupling cushion has the reference number 3.

The shock wave focus as symbolized in Figure 1 is referenced with reference number 4. Within a patient 5, a target organ 6 comprises the target 7, such as a urinary stone.

An imaging ultrasound transducer 8 is enclosed in relationship to the patient 5, and its target 7, as well as the therapy source 2. The imaging ultrasound transducer is operated with an ultrasound scanner 9 which is configured to scan the area 6' in the patient 5. The ultrasound scanner 9 is connected to a monitor 10 which is configured to display an ultrasound image showing the position of the target 7.

A respiration sensor 12 is connected to a respiration detector 13 to acquire information concerning the breathing of the patient and forward it to the control unit 17.

By a function generator 16, either manually or fully automatically, a predefined movement pattern is selected.

For manual selection, a user interface 14, such as a keyboard, is configured to select a pattern function, defining respective parameters. The selected movement pattern is stored in a storage device 15.

In a preferred embodiment, the function generator 16 receives the breathing signal to select a predefined movement pattern. The selection can be conducted manually or, in a preferred embodiment, by the function generator 16 automatically.

The function generator 16 is connected to the control unit 17 such as a motion control. The selected movement pattern is forwarded to the control unit 17.

The control unit 17 is configured to control the movement of the target 7 relative to the emitted shock wave focus 4. Figure 1 discloses the embodiment where the target is moved by stretcher 11 using the mechanical drive unit 18.

The patient is put on the patient stretcher and an ultrasound image, with the help of the imaging ultrasound transducer 8 and the monitor 10, is displayed to a user of the shock wave therapy device 1.

Depending on the selected predefined movement pattern, the patient stretcher is moved in a combination of three directions in the space, in order to move the target relative to the shock wave focus. If the movement pattern has been carefully selected based on the actual detected respiration of the patient on the patient stretcher 11, a high probability of correct movement of the target, in view of keeping the target 7 in the shock wave, is achieved.

The user can monitor and optimize the compensation of the respiratory motion by the imaging modality 8,9,10.

Such, a reliable, safe and cost efficient shock wave therapy device is presented.

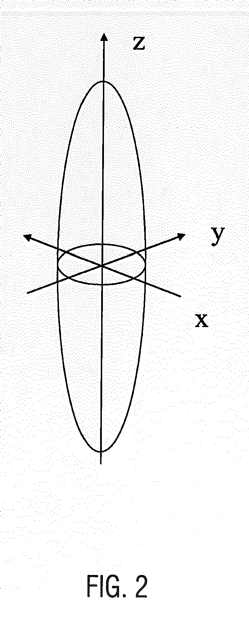

In Figure 2 a typical ellipsoidal geometry of -6 dB extracorporeal shockwave lithotripsy focus is discernible. The focus extension is longer in the axial direction, which is parallel to the shockwave penetration than in the lateral axes. Those lateral axes are x and y and define a lateral plane.

Since the shock waves cannot be transmitted along the longitudinal cranio-caudal patient axis, but perpendicular to it, the cranio-caudal movement of the kidney is approximately within the lateral plane. The displacement due to respiratory motion are typically longer than the -6 dB lateral focal widths of today's shock wave therapy devices.

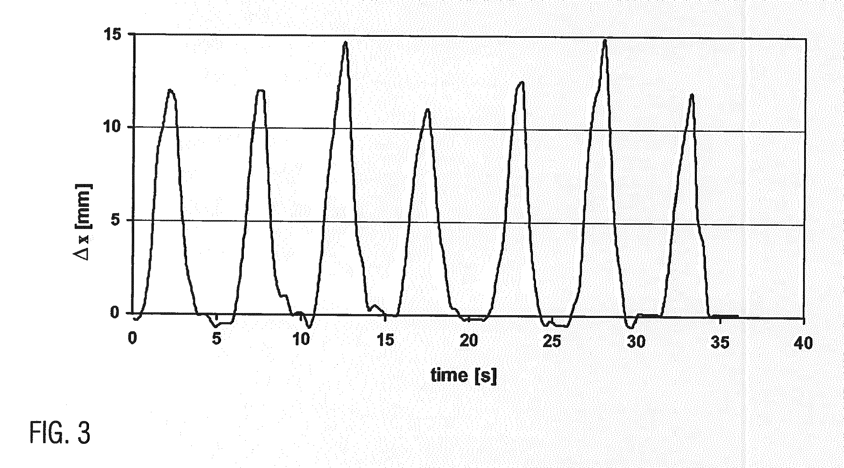

In Figure 3, the respiratory motion of a target such as a urinary stone within the kidney along a lateral axis is displayed as a function of time.

Seven respiratory cycles are mapped together with the thereof caused movement of the target. The amplitude varies between 11.0 mm and 14.8 mm. The duration of each breathing cycle varies between 4.8 seconds and 5.4 seconds.

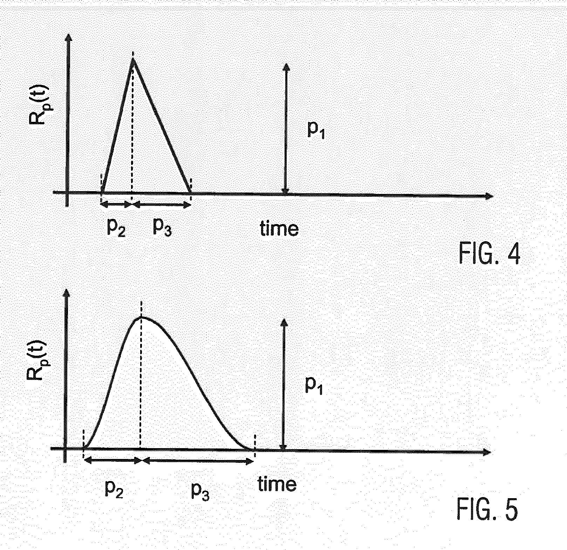

In Figures 4 and 5, examples of pathes of motion caused by the control unit to compensate the breathing related movement of the target. It is the relative movement between the target and the shock wave focus, which is obtained for example by moving the patient stretcher, i.e. the target, or by moving the shockwave focus. The path of motion in Figure 4 is made up by two straight lines.

The progression of the curve can be modelled as follows:

$$R_p(t) = \begin{cases} P_1\left(\dfrac{t}{P_2}\right) & with \quad 0 \le t \le P_2 \\ P_1\left(\dfrac{t - P_2}{P_3}\right) & with \quad P_2 < t \le P_2 + P_3 \end{cases}$$

Figure 5 shows an example of a smooth curve:

$$R_p(t) = \begin{cases} P_1 \cdot \sin^2\left(\dfrac{\pi}{2} \cdot \dfrac{t}{P_2}\right) & with \quad 0 \le t \le P_2 \\ P_1 \cdot \cos^2\left(\dfrac{\pi}{2} \cdot \dfrac{(t - P_2)}{P_3}\right) & with \quad P_2 \le t \le P_2 + P_3 \end{cases}$$

$P_1$ is the height of the amplitude of the componential movement, preferably selectable steplessly or in a satisfactorily subdivided scale between 1 mm and 30 mm. At least the values of 5 mm, 10 mm, 15 mm and 20 mm shall be selectable.

$P_2$ resembles the length of the inhaling movement and shall again be steplessly or adequately subdivided be choosable, so that at least the values of 1, 1.5 and 2 seconds are selectable.

$P_3$ resembles the length of the exhaling movement. Again a stepless selection or an adequately subdivided scale shall be provided so that at least the values of 1,1.5 and 2 seconds and/ or 2.5 seconds are choosable/selectable.

Finally not only the height of the amplitude $P_1$, the length of the inhaling related movement $P_2$ and the length of the expiration related movement $P_3$ shall be choosable, by choosing preselected values but also the form of the movement, i.e., a linear form as in Figure 4 or a smooth curve as in Figure 5.

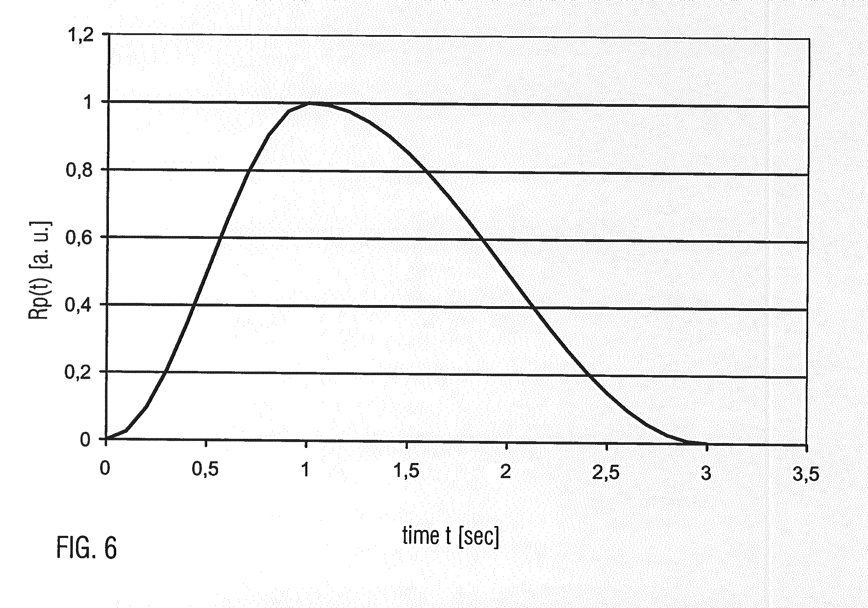

In Figure 6 the compensation movement of Figure 5 is shown in a greater scale. The height of the amplitude $P_1$ is one unit. One unit can be, for example, 10 mm. $P_2$ has been selected to be 1 second, whereas the expiration related movement time $P_3$ is 2 seconds long.

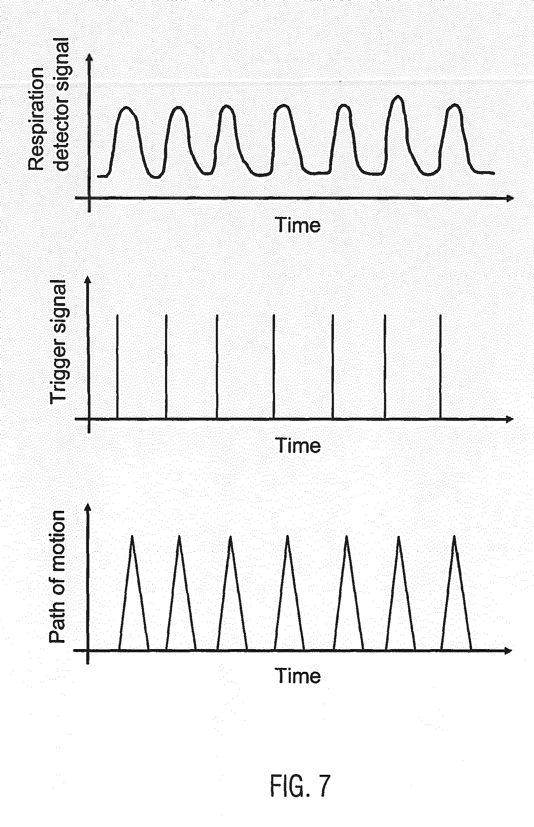

In Figure 7 the respiration signal is shown in the upper part. The trigger signal which is deduced from the respiration signal is shown in the middle part. The path of motion which is composed of a sequence of single curves of the type as shown in Figure 4 is given in the bottom of Figure 7. On the abscissa time is shown, whereas on the ordinate the signal amplitude or the motion amplitude is shown.

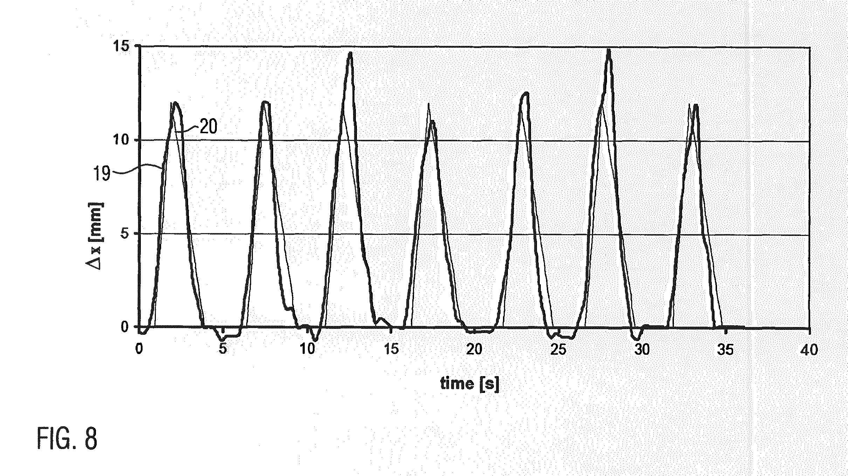

In Figure 8 the amount of displacement is shown on the ordinate, whereas the time is shown on the abscissa. The graph 19 represents the movement of the target due to respiration relative to the shock wave focus shown, whereas the graph 20 shows the relative movement caused by the control unit. The target therefore remains very near to the shock wave focus.

If the relative movement caused by the control unit is realised by moving the patient stretcher, the respiration related movement is in a first direction, the compensation is in a second direction, generally opposed to the first direction. If on the other hand, the relative movement is realised by moving the shock wave focus, the compensation is generally in the same direction as the respiration related movement.

**Claims**

1. A shock wave therapy device (1) with a shock wave therapy source (2) that is configured to emit a shock wave (4) having a focus onto a target (7) in a body of a patient (5),

   with an imaging device (10), which is configured to visualize the target (7),
   with a control unit (17), which is configured to apply movement between the target (7) and the emitted shock wave focus (4),
   with a respiration detector (13), which is configured to detect the respiration of a patient (5) and configured to produce a respiration signal,
   **characterized in that**
   the control unit (17) is configured to control the movement of the target (7) or the shock wave focus according to a preselected movement pattern, and the movement is triggered by the respiration signal of the respiration detector, so that the target remains approximately stable relative to the shock wave focus (4) during the respiratory cycle.

2. The shock wave therapy device (1) of claim 1, **characterized in** comprising a user interface (14), which is configured to select a certain movement pattern.

3. The shock wave therapy device (1) according to claim 1 or 2, **characterized in** comprising a storage device (15) in which one or more certain movement patterns are stored.

4. The shock wave therapy device (1) according to one of claims 1 to 3, **characterized in that** the preselected movement pattern is a predefined movement pattern, based on empirical data, which has been acquired previously to the operation of the shock wave therapy device (1).

5. The shock wave therapy device (1) according to one of claims 1 to 4, **characterized in that** the control unit is configured to cause one or more motors to move such as to move the shock wave therapy source (2) and/or the control is configured unit to cause movement of the focus of the shock wave therapy source by electronic beam steering.

6. The shock wave therapy device (1) according to one of claims 1 to 5, **characterized in** comprising a function generator (16), which is configured to select a certain predefined movement pattern, depending on the respiration signal of the patient (5).

7. The shock wave therapy device (1) according to one of claims 1 to 6, **characterized in that** the height of the amplitude ($P_1$) of the movement of the target (7), and/or the duration of the inhalation related movement ($P_2$), and/or the length of an expiration related movement ($P_3$), and/or the form of the curve of the movement is choosable.

8. The shock wave therapy device (1) according to one of claims 1 to 7, **characterized in** having an override button to choose a predefined movement pattern, resemblying a movement of the target (7), which ensures the least deviation from a movement of the target (7), suitable for a multitude of patients.

9. The shock wave therapy device (1) according to one of claims 6 to 8, **characterized in that** the function generator (16) is configured to select the predefined movement pattern automatically.

10. The shock wave therapy device (1) according to one of claims 1 to 9, **characterized in** the control unit being configured to cause one or more motors (18) to move such as to move a patient stretcher (11).

11. The shock wave therapy device (1) according to one of claims 1 to 10, **characterized in** comprising a monitor (10) suitable to display an ultrasound image of a target area (6) including the target (7).

**EP 2 628 456 B1**

**Patentansprüche**

1. Stoßwellentherapievorrichtung (1) mit einer Stoßwellentherapiequelle (2), die eingerichtet ist, um eine Stoßwelle (4) mit einem Fokus auf ein Ziel (7) in einem Körper eines Patienten (5) zu emittieren,
   mit einer Bildgebungseinrichtung (10), die eingerichtet ist, um das Ziel (7) zu visualisieren,
   mit einer Steuereinheit (17), die eingerichtet ist, um eine Bewegung zwischen dem Ziel (7) und dem emittierten Stoßwellenfokus (4) vorzusehen,
   mit einer Atmungserfassungseinrichtung (13), die eingerichtet ist, um die Atmung eines Patienten (5) zu erfassen, und eingerichtet ist, um ein Atmungssignal zu erzeugen,
   **dadurch gekennzeichnet, dass**
   die Steuereinheit (17) eingerichtet ist, um die Bewegung des Ziels (7) oder des Stoßwellenfokus in Übereinstimmung mit einem vorausgewählten Bewegungsmuster zu steuern, wobei die Bewegung durch das Atmungssignal der Atmungserfassungseinrichtung ausgelöst wird, sodass das Ziel annähernd stabil relativ zu dem Stoßwellenfokus (4) während des Atmungszyklus bleibt.

2. Stoßwellentherapievorrichtung (1) nach Anspruch 1, **gekennzeichnet durch** eine Benutzerschnittstelle (14), die konfiguriert ist, um ein bestimmtes Bewegungsmuster auszuwählen.

3. Stoßwellentherapievorrichtung (1) nach Anspruch 1 oder 2, **gekennzeichnet durch** eine Speichereinrichtung (15), in der eine oder mehrere bestimmte Bewegungsmuster gespeichert sind.

4. Stoßwellentherapievorrichtung (1) nach einem der Ansprüche 1 bis 3, **dadurch gekennzeichnet, dass** das vorausgewählte Bewegungsmuster ein vordefiniertes Bewegungsmuster ist, das auf empirischen Daten basiert, die vor dem Betrieb der Stoßwellentherapievorrichtung (1) erhalten wurden.

5. Stoßwellentherapievorrichtung (1) nach einem der Ansprüche 1 bis 4, **dadurch gekennzeichnet, dass** die Steuereinheit eingerichtet ist, um eine Bewegung eines oder mehrerer Motoren für eine Bewegung der Stoßwellentherapiequelle (2) zu veranlassen, und/oder die Steuereinheit eingerichtet ist, um eine Bewegung des Fokus der Stoßwellentherapiequelle durch eine Elektronenstrahllenkung zu veranlassen.

6. Stoßwellentherapievorrichtung (1) nach einem der Ansprüche 1 bis 5, **gekennzeichnet durch** einen Funktionsgenerator (16), der eingerichtet ist, um ein bestimmtes vordefiniertes Bewegungsmuster in Abhängigkeit von dem Atmungssignal des Patienten (5) auszuwählen.

7. Stoßwellentherapievorrichtung (1) nach einem der Ansprüche 1 bis 6, **dadurch gekennzeichnet, dass** die Höhe der Amplitude ($P_1$) der Bewegung des Ziels (7) und/oder die Dauer der auf die Einatmung bezogenen Bewegung ($P_2$) und/oder die Länge der auf die Ausatmung bezogenen Bewegung ($P_3$) und/oder die Form der Bewegungskurve auswählbar sind.

8. Stoßwellentherapievorrichtung (1) nach einem der Ansprüche 1 bis 7, **gekennzeichnet durch** eine Override-Taste zum Wählen eines vordefinierten Bewegungsmusters, das einer Bewegung des Ziels (7) ähnelt, wodurch die geringste Abweichung von einer Bewegung des Ziels (7) sichergestellt wird, die für eine Vielzahl von Patienten geeignet ist.

9. Stoßwellentherapievorrichtung (1) nach einem der Ansprüche 6 bis 8, **dadurch gekennzeichnet, dass** der Funktionsgenerator (16) eingerichtet ist, um das vordefinierte Bewegungsmuster automatisch auszuwählen.

10. Stoßwellentherapievorrichtung (1) nach einem der Ansprüche 1 bis 9, **dadurch gekennzeichnet, dass** die Steuereinheit eingerichtet ist, um eine Bewegung eines oder mehrerer Motoren für eine Bewegung einer Patientenbahre (11) zu veranlassen.

11. Stoßwellentherapievorrichtung (1) nach einem der Ansprüche 1 bis 10, **gekennzeichnet durch** einen Monitor (10), der geeignet ist zum Anzeigen eines Ultraschallbilds eines Zielbereichs (6) einschließlich des Ziels (7).

**Revendications**

1. Dispositif de thérapie par onde de choc (1) avec une source de thérapie par onde de choc (2) qui est configurée

8

pour émettre une onde de choc (4) ayant un foyer sur une cible (7) dans le corps d'un patient (5),

avec un dispositif d'imagerie (10) qui est configuré pour visualiser la cible (7),

avec une unité de commande (17) qui est configurée pour appliquer un mouvement entre la cible (7) et le foyer de l'onde de choc émise (4),

avec un détecteur de respiration (13) qui est configuré pour détecter la respiration du patient (5) et configuré pour produire un signal de respiration,

**caractérisé en ce que**

l'unité de commande (17) est configurée pour commander le mouvement de la cible (7) ou le foyer de l'onde de choc conformément à un motif de mouvement présélectionné, et le mouvement est déclenché par le signal de respiration du détecteur de respiration, de sorte que la cible reste approximativement stable par rapport au foyer de l'onde de choc (4) pendant le cycle respiratoire.

2. Dispositif de thérapie par onde de choc (1) selon la revendication 1, **caractérisé en ce qu'**il comprend une interface d'utilisateur (14) qui est configurée pour sélectionner un certain motif de mouvement.

3. Dispositif de thérapie par onde de choc (1) selon la revendication 1 ou 2, **caractérisé en ce qu'**il comprend un dispositif de mémorisation (15) dans lequel sont mémorisés un ou plusieurs motifs de mouvement.

4. Dispositif de thérapie par onde de choc (1) selon l'une des revendications 1 à 3, **caractérisé en ce que** le motif de mouvement présélectionné est un motif de mouvement prédéfini, fondé sur des données empiriques qui ont été acquises préalablement au fonctionnement du dispositif de thérapie par onde de choc (1).

5. Dispositif de thérapie par onde de choc (1) selon l'une des revendications 1 à 4, **caractérisé en ce que** l'unité de commande est configurée pour faire déplacer la source de thérapie par onde de choc (2) par un ou plusieurs moteurs et/ou l'unité de commande est configurée pour provoquer le mouvement du foyer de la source de thérapie par onde de choc par orientation d'un faisceau électronique.

6. Dispositif de thérapie par onde de choc (1) selon l'une des revendications 1 à 5, **caractérisé en ce qu'**il comprend un générateur de fonction (16) qui est configuré pour sélectionner un certain motif de mouvement prédéfini, en fonction du signal de respiration du patient (5).

7. Dispositif de thérapie par onde de choc (1) selon l'une des revendications 1 à 6, **caractérisé en ce que** la hauteur de l'amplitude ($P_1$) du mouvement de la cible (7), et/ou la durée du mouvement associé à l'inhalation ($P_2$), et/ou la longueur du mouvement associé à l'expiration ($P_3$), et/ou la forme de la courbe du mouvement peuvent être choisies.

8. Dispositif de thérapie par onde de choc (1) selon l'une des revendications 1 à 7, **caractérisé en ce qu'**il comporte un bouton de priorité pour choisir un motif de mouvement prédéfini, ressemblant à un mouvement de la cible (7), qui garantit que le dernier écart par rapport à un mouvement de la cible (7) convient pour une multitude de patients.

9. Dispositif de thérapie par onde de choc (1) selon l'une des revendications 6 à 8, **caractérisé en ce que** le générateur de fonction (16) est configuré pour sélectionner automatiquement le motif de mouvement prédéfini.

10. Dispositif de thérapie par onde de choc (1) selon l'une des revendications 1 à 9, **caractérisé en ce que** l'unité de commande est configurée pour animer un ou plusieurs moteurs (18) de façon à déplacer un brancard de patient (11).

11. Dispositif de thérapie par onde de choc (1) selon l'une des revendications 1 à 10, **caractérisé en ce qu'**il comprend un moniteur (10) approprié pour afficher une image ultrasonore d'une zone cible (6) incluant la cible (7).

FIG. 1

FIG. 2

EP 2 628 456 B1

FIG. 3

FIG. 4

FIG. 5

FIG. 6

FIG. 7

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DE 3915384 C2
- DE 3146628 C3
- DE 10260594 A1
- DE 3943644 C2
- DE 3811872 A1
- EP 0460536 B1
- US 20030149352 A1
- DE 4241161 A1
- DE 3900893 A1
- DE 3736733 A1
- DE 3743883 C3
- DE 102007004334 A1
- DE 3621935 C2
- US 5065741 A

### Non-patent literature cited in the description

- **C.BOHRIS et al.** Ultrasound Monitoring of Kidney Stone Extracorporeal Shockwave Lithrotripsy with an with an External Transducer: Does Fatty Tissue Cause Image Distortions That Affect Stone Comminution?. *J. Endourology,* 2010, vol. 24, 81-88
- **DAVIS S. et al.** Ultrasound quantitation of respiratory organ motion in the upper abdomen. *J.Br. Radiol,* vol. 67, 1096-1102
- **L. SCHWARTZ et al.** Kidney mobility during respiration. *Radiotherapy and Oncology,* 1994, vol. 32, 84-86
- **C. BOHRIS.** *J. Endourology,* 2010, vol. 24, 81-88